(19) Europäisches Patentamt / European Patent Office / Office européen des brevets

(11) EP 3 346 975 B1

(12) **EUROPEAN PATENT SPECIFICATION**

(45) Date of publication and mention of the grant of the patent:
**20.05.2020 Bulletin 2020/21**

(21) Application number: **16709945.6**

(22) Date of filing: **15.03.2016**

(51) Int Cl.:
***A61K 8/33*** (2006.01) ***A61K 8/362*** (2006.01)
***A61K 8/365*** (2006.01) ***A61K 8/368*** (2006.01)
***A61Q 5/04*** (2006.01) ***A61Q 5/06*** (2006.01)
***A61Q 5/10*** (2006.01) ***A61K 8/22*** (2006.01)

(86) International application number:
**PCT/EP2016/055583**

(87) International publication number:
**WO 2017/041904 (16.03.2017 Gazette 2017/11)**

(54) **PROCESS FOR SEMIPERMANENT STRAIGHTENING AND DYEING HAIR**

VERFAHREN ZUM SEMIPERMANENTEN GLÄTTEN UND FÄRBEN DER HAARE

PROCÉDÉ DE DÉFRISAGE SEMI-PERMANENT ET DE COLORATION DE CHEVEUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.09.2015 PCT/EP2015/184309**

(43) Date of publication of application:
**18.07.2018 Bulletin 2018/29**

(73) Proprietor: **Kao Germany GmbH**
**64297 Darmstadt (DE)**

(72) Inventors:
- **NÖCKER, Bernd**
  **64297 Darmstadt (DE)**
- **BREAKSPEAR, Steven**
  **64297 Darmstadt (DE)**
- **BAUER, Peter**
  **64297 Darmstadt (DE)**
- **WOOD, Jonathan**
  **64297 Darmstadt (DE)**

(56) References cited:
**WO-A1-2014/067702** **WO-A2-2014/068102**
**US-A1- 2012 118 316**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 346 975 B1

## Description

The present invention relates to a process for semi-permanent straightening and dyeing hair for improved and milder semi-permanent straightening and dyeing of hair, especially human hair.

Known methods of hair straightening are first of all classified as permanent and semi-permanent hair straightening methods. The permanent straightening method involves application of reducing agents and after breakage of the disulfide bonds and putting hair physically into the straight shape, the disulfide bonds are reformed by mildly oxidizing the hair. The semi-permanent methods involve the use of hair straightening irons on wet hair. Recently, a new method has been made available based on application of an aqueous solution of glyoxylic acid in a strongly acidic pH range in combination with ironing the hair. Such a method delivers long lasting hair straightening. The details of the method are disclosed in various patent applications from Kao Corporation which are published as international applications such as WO 2011/104282 and WO 2012/010351.

Oxidative dyeing of human hair has been used for many decades in order to permanently change the hair color. It involves application of a strongly oxidative composition comprising the dyestuff precursors and, optionally, coupling substances onto hair and leaving it for a certain period of time, usually at elevated temperatures, in order to allow for penetration of the relatively small uncolored dye precursors into the hair. In combination with the action of strong oxidizing agents, the dye precursors polymerize to form larger molecules so that they may not be easily eluted from the hair fiber. At the same time, the effect of the oxidizing agent is to lighten the hair color to provide a relatively homogeneous dyeing base. Since the process involves the use of strong oxidative compositions, the hair fiber itself is negatively affected by such a treatment and consequently loses certain natural cosmetic properties, such as its strength against breaking, its natural elasticity, its natural shine and natural soft feel upon touching.

Moreover, the to be dyed hair is not always homogeneous in its physicochemical status as it may be damaged due to previous chemical treatments, such as dyeing and permanently shaping and/or environmental effects. This often leads to inhomogeneous dyeing performance and, therefore, consumers' dissatisfaction. There is, therefore,a great need for milder and more effective dyeing compositions which overcomes one or more of the above mentioned problems.

Recently in a series of patent applications (US2015/0034119, US2015/0037270, WO2015/017768) methods are published which claim benefits of the combined use of a bismaleate based binding agent in hair chemical treatments such as oxidative hair dyeing, permanently shaping and bleaching for improving of hair structure. The publications are silent on the core of the present invention.

In case the semi-permanently straightened hair is dyed with an oxidative dyeing composition, the dyeing results are not satisfactory and, therefore, the two processes are mostly carried out during two separate hairdresser visits which is time consuming and not economical.

Documents WO 2014/068102 and WO 2014/067702 disclose processes for dyeing and straightening the hair in two steps. The methods of said documents use a first composition (straightening composition) comprising at least one carboxylic acid, and a second composition (dye composition) comprising at least one hair dye, optionally at least one alkalizing agent and optionally at least one oxidizing agent. In the process of the second document, the hair is treated with the said compositions successively, so that the dyeing treatment is performed directly after the straightening treatment. However said documents do not disclose the use of a mixture of one or more carboxylic acids having 3 or more carboxyl groups and/or their salts, with one or more additional acids and/or their salts selected from organic acids having one or two carboxyl groups.

After a long research and careful considerations of the consumers' needs, the inventors of the present invention have unexpectedly found out that when commonly used oxidative dyeing compositions are mixed with another composition comprising predominantly carboxylic acids, the dyeing effect of the composition is improved, long lasting dyeing is achieved, homogeneous dyeing of hair fibers is achieved and natural cosmetic properties of hair is maintained and hair may be semi-permanently straightened before or after oxidative dyeing the hair.

Therefore, the first object of the present invention is a process for semi-permanent straightening and dyeing hair: wherein the semi-permanent straightening comprises the steps of:

a. optionally washing the hair with a cleansing composition and towel drying,

b. applying to the hair a treatment composition A comprising at least one compound of the general structure

R-CO-R' Formula (I)

and/or a hydrate thereof and/or a salt thereof,
wherein R is selected from hydrogen, COOH, CN, optionally substituted $C_1$-$C_{10}$ alkyl, optionally substituted $C_2$-$C_{10}$ alkenyl, optionally substituted $C_2$-$C_{10}$ alkynyl, optionally substituted $C_3$-$C_{10}$ cycloalkyl, optionally substituted $C_6$-$C_{10}$ aryl or a 5-10-membered, optionally substituted heteroaryl group, wherein the optional substituents of the alkyl group

are selected from halogen, hydroxyl, amino and $C_1$-$C_4$ alkoxy, and the optional substituents of the other groups are selected from halogen, hydroxyl, amino, $C_1$-$C_4$ alkyl and $C_1$-$C_4$ alkoxy, and R' is H or COOH, wherein the composition has a pH in the range of 1 to 4 when R' is COOH and in the range of 1 to 6 when R' is H,

c. leaving the composition A on the hair for 1 min to 90 min,

d. optionally rinsing off the hair,

e. optionally drying the hair,

f. heating the hair to a temperature in the range of 130°C to 230°C,

g. optionally rinsing the hair,

wherein the subsequent dyeing process carried out immediately after the steps a to g comprises the steps of:

h. optionally washing, towel drying the hair and optionally drying the hair,

i. applying a ready-to-use composition onto the hair consisting of compositions B, C, and D, which are kept separately until application and mixed to a ready-to-use composition prior to application onto hair at a weight ratio in the range of 1:2:0.1 to 1:1:1 and left on the hair for 1 min to 45 min, and

j. rinsing off the hair with water and/or optionally shampooing the hair, and

k. optionally drying the hair
wherein composition B is an alkaline aqueous composition comprising one or more hair dyes, one or more alkalizing agents and has a pH between 7.5 and 12,
wherein the composition C is an acidic composition having a pH of 2 to 5 and comprising one or more oxidizing compounds, preferably hydrogen peroxide, and
wherein the composition D is a composition comprising

1) one or more carboxylic acids having three or more carboxyl groups and/or their salts, and

2) one or more additional organic acid and/or their salts having one or two carboxyl groups,

wherein composition D comprises the acids of 1) and 2) and/or their salts at a total concentration of 10% to 100% by weight, calculated to the total of composition D,
wherein the ready-to-use composition has a pH in the range of 6.5 to 12 and comprises the acids and/or their salts at a total concentration in the range of 1.0% to 10.0% by weight calculated to the total of the ready-to-use composition.

The second object is a kit for hair, especially human hair, comprising the compositions A, B, C and D as defined above.

The composition A comprises at least one compound according to the general structure

R-CO-R' Formula (I)

wherein R is selected from hydrogen, COOH, CN, optionally substituted $C_1$-$C_{10}$ alkyl, optionally substituted $C_2$-$C_{10}$ alkenyl, optionally substituted $C_2$-$C_{10}$ alkynyl, optionally substituted $C_3$-$C_{10}$ cycloalkyl, optionally substituted $C_6$-$C_{10}$ aryl or a 5-10-membered, optionally substituted heteroaryl group, wherein the optional substituents of the alkyl group are selected from halogen, hydroxyl, amino and $C_1$-$C_4$ alkoxy, and the optional substituents of the other groups are selected from halogen, hydroxyl, amino, $C_1$-$C_4$ alkyl and $C_1$-$C_4$ alkoxy and R' is COOH or H.

The preferred carboxylic acids are glyoxylic acid, pyruvic acid and 2-ketobutyric acid and the preferred aldehyde is formaldehyde.

The carboxylic acid according to the Formula (I) may be comprised in the composition in its free acid form. The carbonyl group adjacent to the carboxyl group of the acid may also be present in the hydrate form. Apart from the free acid form and the hydrate thereof, salts of the acid or the hydrate may also be used.

The hydrate of the acid of Formula (I) may be formed when providing the composition as an aqueous solution. For instance, glyoxylic acid (H-CO-COOH) in aqueous solution is almost quantitatively present as the hydrate (H-

$C(OH)_2$-COOH). Besides, the hydrate may also condense to dimers.

A salt of the carboxylic acid of Formula (I) may also be used. As examples, alkali metal salts such as the sodium or potassium salt, alkaline earth metal salts such as the magnesium salt or the calcium salt and ammonium salts may be mentioned.

In the present invention, glyoxylic acid and/or a hydrate thereof and/or salts thereof is the most preferred compound of Formula (I).

The concentration of the at least one compound according to the general structure of the formula (I) and/or a hydrate thereof and/or salts thereof is in the range of 0.1% to 40%, preferably 0.5% to 30%, more preferably 1% to 25% and even more preferably 2.5% to 20% by weight, calculated to the total of the composition A.

The pH of the composition A is in the range of 1 to 4 when the R' is COOH, preferably in the range of 1 to 3, more preferably 1 to 2.5, as measured directly and at ambient temperature (25°C) and in the range of 1 to 6 when R' is H. The pH of the compositions may be adjusted using known alkaline solutions, preferably with sodium hydroxide solution.

Conventional hair shaping/straightening techniques are based on the reorganization of the disulfide bridges and involve a cleavage of the disulfide bonds by using a sulfur-based reducing agent, followed by the shaping of the hair and the formation of new disulfide bonds by the action of an oxidizing agent. In contrast, the present invention does not utilize cleavage of the disulfide bonds and fixing the bonds in the new shape. Therefore, the straightening composition of the present invention does not require the presence of sulfur-based reducing agents. However, up to 2% by weight calculated to the total of the composition sulfur based reducing agents does not interfere with the straightening performance of the compositions. Therefore, the treatment composition has less than 2% by weight of sulfur-based reducing agents, and preferably is free of sulfur-based reducing agents.

After application of the composition A, it is left on the hair for a period of 1 min to 90 min and optionally rinsed off and the hair is optionally dried. Subsequently the hair is heated up to a temperature in the range of 130°C to 230°C, using an iron, preferably a flat iron. When using the flat iron for heating the hair, the number of passes through the length of the hair may be determined depending on the degree of frizziness and/or curliness of hair. Typically, 2 to 8 passes through the hair should deliver a satisfactory straightening effect.

The composition B comprises one or more hair dyes. Suitably, the composition B comprises one or more oxidative dye precursors and optionally one or more coupling substances.

Suitable non-limiting examples of oxidative dye precursor classes are p-phenylendiamines, p-aminophenols, and heterocyclic compounds such as diaminopyrazols and substituted pyrimidines, and suitable coupling substances are resorcinols, m-aminophenols, m-phenylendiamines, pyridines, and naphthols.

Non-limiting examples of the oxidative dye precursor compounds are p-phenylenediamine, p-aminophenol, 2,5-diamino-toluene, 2-n-propyl or 2-ethyl-p-phenylenediamine, 2,6-di-methyl-p-phenylene- diamine, 2-(2,5-diaminophenyl) ethanol, 1-amino-4-bis-(2'-hydroxy-ethyl)amino- benzene, 2-(2-hydroxyethyl amino)-5-aminotoluene, 4,4'-diaminodiphenylamine, 4-aminodiphenylamine, 2-amino-5-N,N-diethyl aminotoluene, 4-amino-N-ethyl-N-isopropyl aniline, 2-chloro-p-phenylenediamine, 1-β-hydroxyethyl-2,5-diamino-4-chlorobenzene, 1-β-hydroxyethyl-2,5-diamino-4-methyl benzene, 2-methoxy-p-phenylenediamine, N,N-diethyl-p-phenylenediamine, 1-amino-4-β-methoxyethyl aminobenzene, 1-dimethyl-amino-4-aminobenzene, 1-hydroxy-2,5-diamino-4-methyl benzene, 1-hydroxymethyl-2,5-diaminobenzene, 1,3-dimethyl-2,5-diaminobenzene, 1,4-diamino isopropyl benzene and/or 1-amino-4-β-hydroxypropyl aminobenzene, 1-hydroxyethyl-4,5-diaminopyrazole, 3,4-diamino-5-hydroxypyrazole, 3,5-diaminopyrazole, 3,5-diamino pyrazol-1-carboxamide, 3-amino-5-hydroxypyrazole, 1-phenyl-2-methylpyrazole, 1-phenyl-3-methylpyrazole-5-one, 3,5-dimethylpyrazole, 3,5-dimethylpyrazole-1-methanol, 3,5-diamino-1,2,4-triazole, 4-aminophenol and the derivatives thereof such as 4-amino-3-methylphenol, 2-chloro-4-aminophenol, 2,6-dichloro-4-aminophenol, 2,4-diamino-phenol, 2,6-dibromo-4-aminophenol, tetraamino pyrimidines, triaminohydroxy pyrimidines, diaminomono- and -dihydroxy pyrimidines, aminotriazines, 5-amino salicylic acid and/or 1,2,4-triamino benzene, 2,5-diaminopyridine, 2,3-diaminopyridine, 2,6-diaminopyridine, 3-amino-2-methyl amino-6-methoxypyridine, 2-dimethyl-5-aminopyridine, 2-dimethyl aminoethyl-3-hydroxypyridine, 2-amino-4,6-dimethyl pyridine, 2-amino-3-hydroxypyridine, 3-amino-2(β-hydroxyethyl amino)-6-methoxy pyridine, 2,6-dimethyl amino-5-aminopyridine, 2-di(hydroxyethyl) amino-5-aminopyridine, 2-hydroxyethyl amino-5-aminopyridine, 4-hydroxy-2,5,6-triaminopyrimidine and/or the water-soluble salts thereof, and mixture thereof.

The total concentration of the dye precursors (developing substances) customarily ranges between 0.001 % to 5%, preferably 0.01% to 4% and more preferably 0.05% to 3%, and most preferably 0.1% to 2% by weight, calculated to the total of the composition A.

The suitable non-limiting examples of the coupling substance if present in the composition A are 5-amino-2-methylphenol, 2-methyl-5-hydroxyethylaminophenol, 2,4,-diaminophenoxyethanol, 2-amino-4-hydroxyethylaminoanisol, 2-methyl-5-amino-6-chlorphenol, 1,3-bis(2,4-diaminophenoxy)propane, 2-bis-(2-hydroxyethyl)-aminotoluene, 2-amino-5-methylphenol, resorcinol, 2-methyl-resorcinol, 4-chlororesorcinol, 2-amino-4-chlorophenol, 5-amino-4-methoxy-2-methylphenol, 2-aminophenol, 3-amino-phenol, 1-methyl-2-hydroxy-4-aminobenzene, 3-N,N-dimethyl aminophenol, 2,6-dihydroxy-3,5-dimethoxypyridine, 5-amino-3-methylphenol, 6-amino-3-methylphenol, 1,3-diamino- benzene, 1-amino-3-(2'-hydroxyethylamino)benzene, 1-amino-3-[bis(2'-hydroxy-ethyl) amino]benzene, α-naphthol, 4,6-dichlororesorcinol, 1,3-diamino-toluene, 4-hydroxy-1,2-methylenedioxy benzene, 1,5-dihydroxy naphthalene, 1,6-dihydroxy naphthalene, 1,7-dihydroxy naphthalene, 2,7-dihydroxy naphthalene, 1-hydroxy-2-methyl naphthalene, 4-hydroxy-1,2-methyldioxy benzene, 2,4-diamino-3-chlorophenol, 5-amino-2-methoxyphenol and/or 1-methoxy-2-amino-4-(2'-hydroxyethyl amino)benzene or the water-soluble salts thereof and mixture thereof.

In the composition B the coupling substance(s) as reaction partners of the developing substance(s) are present in approximately the same molecular proportions as the developing substances, i.e., at a total concentration in the range of 0.001 % to 5%, preferably 0.01% to 4% and more preferably 0.05% to 3%, and most preferably 0.1% to 2% by weight, calculated to the total of the composition B.

The composition B comprises furthermore one or more alkalizing agents, preferably selected from ammonia, alkyl- or alkanolamines according to the general structure

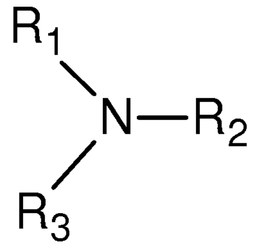

wherein $R_1$, $R_2$, and $R_3$ are same or different H, from $C_1$ to $C_4$, $C_3$ to $C_4$ unsaturated alkyl, $C_3$ to $C_4$ branched alkyl, $C_1$ to $C_4$ hydroxyl alkyl, $C_3$ to $C_4$ unsaturated hydroxyl alkyl, $C_3$ to $C_4$ branched hydroxyl alkyl, with the condition that at least one of $R_1$, $R_2$, or $R_3$ is different from H, wherein the alkalizing agents preferably selected from ammonia, monoethanolamine, and aminomethylpropanol, and particularly suitable one is aminomethylpropanol.

The alkalizing agent is comprised in the composition B at a total concentration of 1% to 20%, preferably 1% to 17.5%, more preferably 2% to 15% and most preferably 2.5% to 13% by weight calculated to the total of the composition B.

The pH of the composition B is in the range of 7.5 to 12, preferably 9 to 11, more preferably 9 to 10.5 and most preferably 9.5 to 10.5.

The composition C is an aqueous composition and comprises one or more oxidizing agent(s). The oxidizing agents suitable are hydrogen peroxide, urea peroxide, melamin peroxide or perborate salts. The most preferred is hydrogen peroxide. The composition C comprises one or more oxidizing agents at a total concentration of 1% to 20% by weight, preferably 2% to 15%, more preferably 2% to 12% and most preferably 3% to 12% by weight, calculated to total of composition C. The composition C may be in the form of a solution, thickened gel or an emulsion. Emulsion form is particularly preferred.

The pH of the composition C is in the range of 2 to 5, preferably 2.5 to 4.5, more preferably 2.5 to 4.

The composition D comprises

1) one or more carboxylic acids having three or more carboxyl groups and/or their salts, and
2) one or more additional organic acid and/or their salts having one or two carboxyl groups.

Suitable carboxylic acids with three or more carboxyl groups and/or their salts are citric acid, ethylenediamine tetraacetic acid (EDTA), pyromellitic acid and glutamate diacetate. The ethylenediamine tetraacetic acid (EDTA) and/or its salts such a monosodium, disodium, trisodium and tetrasodium salts are the most preferred ones.

Suitable organic acids with one or two carboxyl groups and/or their salts are acetic acid, malic acid, lactic acid, glycolic acid, tartaric acid, formic acid, oxalic acid, malonic acid, succinic acid, glutaric acid, adipic acid, maleic acid, and fumaric acid. In the present invention the composition D comprises as the second acid one or more organic acids having one or two carboxyl groups and the most preferred acid is malic acid and/or its salts such as sodium, potassium and ammonium salts.

The composition D comprises the two acids at a total concentration in the range of 10% to 100% by weight, preferably 12.5% to 90%, more preferably 12.5% to 75% by weight and most preferably 12.5% to 60% by weight, calculated to the total of composition D.

The two acids are comprised in the composition D at a weight ratio of first acid (1) to second acid (2) in the range from 1:0.1 to 1:250, preferably from 1:0.2 to 1:150, and more preferably from 1:0.5 to 1:100 and most preferably 1:50.

The composition D may be in the form of a powder, a dispersion, an emulsion or a solution. In a preferred embodiment of the present invention the composition D is an aqueous composition and preferably has a pH in the range of 1 to 5, preferably 2 to 4, more preferably in the range of 2.5 to 3.6. In the case that the pH must be adjusted to a certain value, the composition D comprises one or more alkalizing agents preferably selected from ammonia, alkyl- or alkanolamines according to the general structure disclosed above. Particularly preferred alkalizing agent is aminomethylpropanol.

The alkalizing agent is comprised in the composition D at a total concentration of 1% to 20%, preferably 1% to 17.5%, more preferably 2% to 15% and most preferably 2.5% to 13% by weight calculated to the total of the composition D.

In a further preferred embodiment of the present invention, the composition D comprises one or more thickening polymers selected from anionic, nonionic, cationic and amphoteric polymers, preferably selected from polymers with a viscosity of at least 500 mPa·s measured at a polymer concentration of 1% by weight in water and at 20°C with a Brookfield viscometer, such as at 10 rpm for 1 minute, with an appropriate spindle.

Suitable polymers are cellulose polymers, alginates, polysaccarides and acrylic acid polymers, preferably methyl cellulose, ethyl cellulose, hydroxyethylcellulose, methylhydroxyethylcellulose, methylhydroxypropylcellulose, carboxymethyl cellulose, alginic acids, sodium alginates, ammonium alginates, calcium alginates, gum arabic, guar gum or xanthan gum, dehydroxanthan gum or acrylic acid polymers known with the CTFA adopted name Carbomer and its derivatives.

The preferred polymers are dehydroxanthan gum, xanthan gum, and polymeric anionic thickeners Carbomer and its derivatives. The particularly preferred thickening agent is dehydroxanthan gum. The thickening agents are preferably comprised in the composition D at a total concentration in the range of 0.1% to 5%, preferably, 0.2% to 3%, more preferably 0.25% to 2.5% and most preferably 0.3% to 2% by weight calculated to the total of the composition D.

In another preferred embodiment of the present invention the composition B and/or C and/or D comprise(s) one or more hair direct dyes. Suitable ones are cationic, anionic and nitro dyes. Plant dyes are also suitable for the compositions of the present invention.

Suitable anionic direct dyes are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9, Disperse Violet 1, HC Blue 18, HC Red 18 and HC Yellow 16 and their alkali metal salts such as sodium, potassium. Among those, the most preferred anionic dyestuffs are Acid Red 52, DC Violet 2, DC Red 33, DC Orange 4, DC Red 27, DC Yellow 10, HC Blue 18, HC Red 18 and HC Yellow 16.

Suitable cationic dyes are in principle those available on the market for cosmetic hair colouring applications. For this purpose, special reference is made to the PCT application WO 95/15144 of Ciba-Geigy AG. Some examples to those are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 76, Basic Violet 1, Basic Violet 2, Basic Violet 3, Basic Violet 10, Basic Violet 14, Basic Yellow 57, Basic Red 51, Basic Yellow 87 Basic Orange 31, and HC Blue 17. The most preferred ones are Basic Red 51, Basic Yellow 87 and Basic Orange 31 sold by BASF, and HC Blue 17.

Suitable nitro dyes are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Plant dyestuffs can also be used alone or in combination with synthetic direct-acting dyestuffs, for example henna (red or black), alkanna root, laccaic acid, indigo, logwood powder, madder root and rhubarb powder, etc.

The composition B and/or C and/or D may comprise one or more hair direct dye at a total concentration of 0.01% to 10%, preferably 0.05% to 7.5% and more preferably 0.1% to 5% by weight calculated to the total of the compositions B and/or C and/or D. The compositions can also comprise mixtures of several direct dyes i.e. an anionic, a cationic and/or a nonionic ones. In such a case the dyes may be mixed at any ratio with each other.

Any of the compositions A, B, C or D may comprise one or more of the commonly used hair conditioning compounds. These compounds are for example fatty alcohols, surfactants such as anionic, nonionic, cationic and amphoteric ones, ubiquinones, ceramides, organic solvents, lipophilic ingredients such as vegetable oils, mineral oils, silicones, fatty acid fatty alcohol esters, preservatives, amino acids, and polyols. It should be noted that these compounds are optionally comprised in the any of the compositions and their incompatibility must be carefully considered prior to addition in the compositions.

Any of the compositions may comprise one or more fatty alcohols. In particular the compositions A, B, C, and/or

D may be aqueous composition and may further be in the form of an emulsion and then comprises preferably one or more fatty alcohols. Suitable fatty alcohols are the ones with the chain length of 14 to 22 C atoms which may be saturated or unsaturated, linear or branched which may as well be substituted. Non-limiting examples are myristyl alcohol, cetyl alcohol, stearyl alcohol, behenyl alcohol and cetostearyl alcohol.

The total concentration of fatty alcohol is in the range from 0.1% to 20%, preferably 1% to 15% by weight, calculated to the total of each composition.

Compositions according to the present invention may comprise surfactants selected from anionic, nonionic, amphoteric and/or cationic surfactants. The anionic, nonionic, amphoteric surfactants are used generally as emulsifier or solubilizer, whereas the cationic surfactants are at the same time particularly used as hair conditioners.

Anionic surfactants suitable are in principle known from the cleansing compositions. These are anionic surfactants of the sulfate, sulfonate, carboxylate and alkyl phosphate type, for example, the known $C_{10}$-$C_{18}$-alkyl sulfates, and in particular the respective ether sulfates, for example, $C_{12}$-$C_{14}$-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfation of fatty acid alkanolamides, and the alkali salts thereof, as well as the salts of long-chain mono- and dialkyl phosphates.

Additional anionic surfactants useful within the scope of the invention are $\alpha$-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof as well as alkyl amido polyether carboxylic acids and salts thereof. Such products have been known for some time and are on the market, for example, under the trade name "AKYPO®" and "AKYPO-SOFT®".

Also useful are $C_8$-$C_{20}$-acyl isethionates, alone or in admixture with other anionic surfactants, as well as sulfofatty acids and the esters thereof.

Further suitable anionic surfactants are also $C_8$-$C_{22}$-acyl aminocarboxylic acids or the water-soluble salts thereof. Especially preferred is N-lauroyl glutamate, in particular as sodium salt, as well as, for example, N-lauroyl sarcosinate, N-$C_{12}$-$C_{18}$-acyl asparaginic acid, N-myristoyl sarcosinate, N-oleoyl sarcosinate, N-lauroyl methylalanine, N-lauroyl lysine and N-lauroyl aminopropyl glycine, preferably in form of the water-soluble alkali or ammonium, in particular the sodium salts thereof, preferably in admixture with the above-named anionic surfactants.

It is also possible to use mixtures of several anionic surfactants.

Further suitable surfactants are nonionic surfactants. Non-limiting examples are long-chain fatty acid mono- and dialkanolamides, such as coco fatty acid mono- or diethanolamide and myristic fatty acid mono or diethanolamide, stearic acid mono or diethanolamide, alkyl polyglucosides with an alkyl group of 8 to 18 carbon atoms, and with 1 to 5 glucoside units, sorbitan esters, such as polyethylene glycol sorbitan stearic, palmitic, myristic and lauric acid esters, fatty acid polyglycol esters or polycondensates of ethyleneoxide and propyleneoxide, as they are on the market, for example, under the trade name "Pluronics$^R$", as well as fatty alcohol ethoxylates, $C_{10}$-$C_{22}$-fatty alcohol ethoxylates, known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16": The average degree of ethoxylation thereby ranges between about 2.5 and about 100, preferably about 10 and about 30.

Suitable amphoteric surfactants are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate have also been proven suitable.

Suitable cationic surfactants are according to the general structure

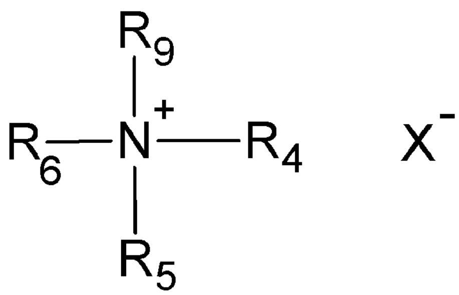

where $R_5$ is a saturated or unsaturated, branched or linear alkyl chain with 8-22 C atoms or

$R_7 CO NH (CH_2)_n$

where $R_7$ is saturated or unsaturated, branched or linear alkyl chain with 7-21 C atoms and n has typical value of 1 - 4 or

$R_8$ CO O $(CH_2)_n$

where $R_8$ is saturated or unsaturated, branched or linear alkyl chain with 7-21 C atoms and n has typical value of 1 - 4, and

$R_4$ is H or unsaturated or saturated, branched or linear alkyl chain with 1 - 22 C atoms or

$R_7$ CO NH $(CH_2)_n$

or

$R_8$ CO O $(CH_2)_n$

where $R_7$, $R_8$ and n are same as above.

$R_9$ and $R_6$ are H or lower alkyl chain with 1 to 4 carbon atoms, and X is typically chloride, bromide, methosulfate.

Typical examples of those ingredients are cetyl trimethyl ammonium chloride, stearyl trimonium chloride, dipalmitoyl dimonium chloride, distearyl dimethyl ammonium chloride, stearamidopropyl trimonuim chloride, dioleoylethyl dimethyl ammonium methosulfate, dioleoylethyl hydroxyethylmonium methosulfate.

The concentration of one or more total surfactants in any of the compositions A, B, C, and/or D is in the range of 0.1% to 20%, preferably 0.2% to 15% and most preferably 0.2% to 10% by weight, calculated to the total of each composition.

The compositions A, B, C, and/or D may further comprise lipophilic ingredients such as vegetable oils, for example, jojoba oil or any other; liquid paraffins, especially paraffinum perliquidum and paraffinum subliquidum; silicones for example linear polysiloxanes such as dimethicones with various consistency and dimethiconols, aminated silicones with primary, secondary, tertiary or quaternary ammonium groups such as amodimethicone, polysilicone 9, and quaternium 80, cyclic silicones such as cyclomethicones, arylated silicones such as phenyl trimethicone; fatty acid esters such as octyl palmitate, isocetyl palmitate, isopropyl palmitate and octyl stearate, $C_{10}$-to $C_{36}$-fatty acid triglycerides, as well as their mixtures. Total concentration of these lipophilic compounds is in the range of 0.1% to 20% by weight, preferably from 1% to 15% by weight, and more preferably from 2% to 10% by weight, calculated to the total of each composition.

Compositions A, B, C, and/or D can also comprise cationic polymers as conditioning and/or thickening agents. Those are cationic cellulose type polymers know as Polymer JR type from Amerchol such as Polyquaternium 10 or cationic guar gum known with trade name Jaguar from Rhône-Poulenc and chemically for example Guar hydroxypropyl trimonium chloride. Furthermore, chitosan and chitin can also be included in the compositions as cationic natural polymers.

Furthermore, it has been found suitable to use those cationic polymers known with their CTFA category name Polyquaternium. Typical examples of those Polyquaternium 4, Polyquaternium 6, Polyquaternium 7, Polyquaternium 10, Polyquaternium 11, Polyquaternium 16, Polyquaternium 22, Polyquaternium 24, Polyquaternium 28, Polyquaternium 30, Polyquaternium 37, Polyquaternium 36, Polyquaternium 46, Polyquaternium 67, and Polyquaternium 72.

Equally suitable polymers are known with their CTFA category name Quaternium are suitable. Those are for example Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-82, Quaternium-83 and Quaternium-84.

The total concentration of cationic polymers may be in the range of 0.1% to 7.5% by weight, preferably 0.3% to 5% by weight and more preferably 0.5% to 2.5% by weight, calculated to the total of each composition.

Compositions A, B, C, and/or D may comprise one or more ceramide compound, such as the one according to general formula

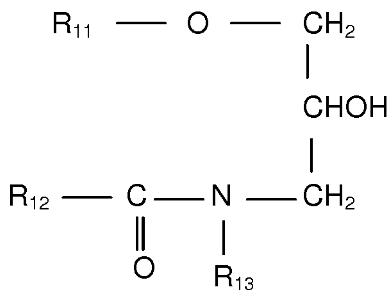

where $R_{11}$ and $R_{12}$ are independent from each other alkyl- or. alkenyl group with 10 to 22 carbon atoms, $R_{13}$ is alkyl or hydroxyl alkyl with 1 to 4 carbon atoms group and n is a number between 1 to 6, preferably 2 or 3. Preferred compound according to the above chemical structure is cetyl-PG-hydroxyethylpalmitamide. The concentration of ceramide type of compounds ranges from 0.01 % to 2%, preferably 0.01 % to 1% by weight calculated to total or each composition.

The compositions A, B, C, and/or D may comprise ubiquinone of the formula:

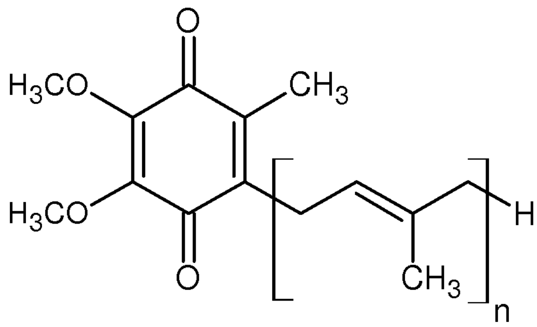

wherein n is a number from 1 to 10. The concentration of ubiquinone can vary between 0.001 % and 10 % by weight, calculated to the total of each composition.

The compositions A, B, C, and/or D may comprise one or more organic solvent such as 2-phenoxyethanol, benzyl alcohol, 2-phenylethanol and 2-benzyloxyethanol. Suitable aliphatic alcohols are ethanol, isopropanol, propanol, n-butanol, isobutanol, t-butanol and 1-pentanol. Concentration of one or more organic solvent is in the range of 0.1 % to 15%, preferably 0.5% to 12.5% and more preferably 1% to 10% and most preferably 1% to 7.5% by weight calculated to the total of each composition.

The compositions A, B, C, and/or D may further comprise one or more amino acids, preferably at a concentration in the range of 0.01% to 5%, preferably 0.1% to 3% and more preferably 0.2% to 2.5% and most preferably 0.25% to 2% by weight calculated to the total of each composition. Suitable ones are all of the known amino acids such as, arginine, alanine, asparagine, glutamine, glycine, histidine, leucine, lysine, methionine, phenylalanine, proline, serine, threonine, tryptophan, tyrosine and valine.

The compositions A, B, C, and/or D may further comprise one or more polyol, preferably at a concentration in the range of 0.01% to 5%, preferably 0.1% to 3% and more preferably 0.2% to 2.5% and most preferably 0.25% to 2% by weight calculated to the total of each composition. Suitable ones are propylene glycol, diproplylene glycol, glycerine, panthenol and its derivatives.

The composition B may further comprise one or more reducing agents for improving long term stability of the oxidative dye precursors. The suitable ones are any of the known reducing agents such as sodium sulfite, ascorbic acid and its salts, thioglycolic acid and its salts. The concentration of the reducing agent in the composition B is typically in the range of 0.1% to 2% by weight calculated to the total of the composition B.

The compositions A, B, C, and/or D may further comprise any known preservatives, if necessary.

The following examples are to illustrate the invention.

## Example 1

**Composition A**

| | % by weight |
|---|---|
| Glyoxylic acid | 12.0 |
| Amodimethicone | 1.0 |
| Hydroxyethylcellulose | 1.5 |
| Sodium hydroxide | q.s. to pH 1.5 |
| Water | to 100 |

**The composition B**

| | % by weight |
|---|---|
| Cetearyl alcohol | 10.0 |
| Cocamide MEA | 4.0 |
| Sodium lauryl sulphate | 1.5 |
| Propylene glycol | 2.0 |
| Cetyltrimonium chloride | 0.5 |
| 2,5,6-Triamino-4-hydroxypyrimidine sulphate | 0.01 |
| 2,5-Diaminotoluene sulphate | 0.55 |
| 4-Chlororesorcinol | 0.17 |
| Resorcinol | 0.05 |
| 3-Aminophenol | 0.03 |
| Sodium sulfite | 1.0 |
| Aminomethylpropanol | 2.0 |
| Ammonium hydroxide | q.s. to pH 10.0 |
| Fragrance, preservative | q.s. |
| Water | to 100 |

**The composition C**

| | | % by weight |
|---|---|---|
| Hydrogen peroxide | | 9.00 |
| Cetyl stearyl alcohol | | 1.70 |
| Phosphoric acid | | q.s. to pH 3.0 |
| Sodium lauryl sulfate | | 0.20 |
| Salicylic acid | | 0.10 |
| Water | ad | 100.00 |

**The composition D**

| | % by weight |
|---|---|
| EDTA tetrasodium salt | 1.0 |
| Malic acid | 13.0 |
| Aminomethylpropanol | 6.0 |
| Hydroxypropyl xanthan gum | 0.6 |
| Cetrimonium chloride | 0.1 |
| Preservative | q.s. |
| Water | to 100 |

The pH of the above composition D was approximately 3.5.

Hair streaks of 25 cm length (3.5 g) were purchased from International Hair Importer & Products, Glendale, NY, USA. The composition A was applied to a shampooed and towel dried hair streak and left on the hair for 15 min at ambient temperature and the hair was dried with a hair drier. Afterwards the hair was treated with a flat iron having a

surface temperature of 180°C. The streak was passed for 3 times with an iron. Afterwards the hair was shampooed with Kerasilk Rich Keratin Care Shampoo and blow dried. Directly after allowing the hair streak to cool down, to hair was applied the ready-to-use composition obtained by mixing the three compositions B, C, and D from above at a weight ratio of B, C and D 1:2:0.2. The resulting composition had a pH of approximately 9.5. The composition was applied onto the streaks of human hair and left on the hair for 30 min at 40°C, then the hair was rinsed off, shampooed with a Dualsenses Color Shampoo and left for air-drying at ambient temperature (inventive process).

For comparative purposes, the same as above was carried out without using the composition D (comparative process). Instead of composition D the same amount of water was added. The resulting composition had a pH of approximately 9.9.

The above disclosed processes were carried out on hair streaks pre-damaged with bleaching using a commercially available bleaching composition under the brand Goldwell.

Color durability was investigated by incubating the hair streaks in a shaking bath with a shaking frequency of 100 $min^{-1}$ for 15 min at 30°C. The water bath was filled with an aqueous solution of sodium laureth sulfate at a concentration of 5% by weight, calculated to the total of the water bath solution. Upon incubation, the hair streaks were rinsed with water and towel dried. Color results were measured prior to incubation and upon incubation by spectrophotometrical analysis with a Datacolor 45G CT instrument delivered from Datacolor Inc., Lawrenceville, NJ, USA. Based on the CIE*Lab color space results obtained by the measurements, $\Delta E_{ab}$ values for color difference were calculated according to equation (1):

$$\Delta E_{ab} = \sqrt{(L_2 - L_1) + (a_2 - a_1) + (b_2 - b_1)} \qquad \text{Equation 1}$$

The $\Delta E_{ab}$ value for hair streaks treated with the inventive process was 5.56, whereas the $\Delta E_{ab}$ value for the hair streaks treated with the comparative process was 9.31. Lower $\Delta E_{ab}$ values correspond to less change in hair color upon incubation and the results clearly showed that the inventive process led to a much lower color change which differs to the comparative process by approximately 4 color units. In conclusion the presented data clearly showed the superior performance of the inventive process compared to the state-of-the-art process.

Similar results were obtained with the following compositions when used with the compositions A and B of the Example 1.

**Example 2**

**The composition D**

| Component | % by weight |
|---|---|
| AMP | 6.0 |
| EDTA tetrasodium salt | 3.0 |
| Malic acid | 13.0 |
| Lactic acid | 4.0 |
| Hydroxypropyl xanthan gum | 0.6 |
| Polyquaternium-10 | 0.1 |
| Water | to 100 |
| pH | 3.4±0.1 |

**Example 3:**

**The composition D**

| Component | % by weight |
|---|---|
| Monoethanolamine (MEA) | 2.7 |

(continued)

| Component | % by weight |
|---|---|
| EDTA tetrasodium salt | 5.0 |
| Malic acid | 15.0 |
| Hydroxypropyl xanthan gum | 0.6 |
| Panthenol | 0.1 |
| Water | To 100 |
| pH | 3.3±0.1 |

**Example 4:**

**The composition D**

| Component | % by weight |
|---|---|
| AMP | 6.0 |
| Citric acid | 5.0 |
| Maleic acid | 15.0 |
| Hydroxypropyl xanthan gum | 0.6 |
| Behenamidopropyl trimonium chloride | 0.2 |
| Water | to 100 |
| pH | 1.5±0.1 |

**Example 5:**

**The composition D**

| Component | % by weight |
|---|---|
| MEA | 2.0 |
| Lactic acid | 15.0 |
| Citric acid | 6.0 |
| Hydroxypropyl xanthan gum | 0.6 |
| Polyquaternium-67 | 0.1 |
| Water | to 100 |
| pH | 2.7±0.1 |

**Example 6**

**The composition D**

| | % by weight |
|---|---|
| EDTA tetrasodium salt | 1.0 |
| Malic acid | 13.0 |

(continued)

| | % by weight |
|---|---|
| Aminomethylpropanol | 6.0 |
| Basic red 51 | 1.00 |
| HC Red 18 | 1.00 |
| Hydroxypropyl xanthan gum | 0.6 |
| Cetrimonium chloride | 0.1 |
| Preservative | q.s. |
| Water | to 100 |

The pH of the above composition was 3.5.

Hair was treated with the composition D from above using the compositions A and B of the Example 1 as described under the Example 1. It was observed that the hair was dyed effectively into intensive red color. Exclusion of the composition D resulted in loss of color brilliance and combability.

**Example 7**

**The composition D (powder)**

| | % by weight |
|---|---|
| EDTA | 7.0 |
| Malic acid | 93.0 |

1 g of the above composition D was added to the mixture of 20 g of composition B and 20 g of composition C (1 to 1) of the Example 1. After mixing thoroughly, the resulting composition was applied onto hair which was already treated with the composition A of the Example 1 and rinsed off after leaving on the hair for 30 min. It was observed that the hair was homogeneously dyed and felt natural upon touching.

**Example 8**

**The composition D**

| | % by weight |
|---|---|
| EDTA monosodium salt | 1.0 |
| Malic acid | 13.0 |
| Aminomethylpropanol | 6.0 |
| Hydroxypropyl xanthan gum | 0.6 |
| Cetrimonium chloride | 0.1 |
| Preservative | q.s. |
| Water | to 100 |

The pH of the above composition D is approximately 3.1.

**Example 9**

**The composition D**

| | % by weight |
|---|---|
| EDTA disodium salt | 1.0 |
| Malic acid | 13.0 |

(continued)

| | % by weight |
|---|---|
| Aminomethylpropanol | 6.0 |
| Hydroxypropyl xanthan gum | 0.6 |
| Cetrimonium chloride | 0.1 |
| Preservative | q.s. |
| Water | to 100 |

The pH of the above composition D is approximately 3.2.

**Example 10**

**The composition D**

| | % by weight |
|---|---|
| EDTA trisodium salt | 1.0 |
| Malic acid | 13.0 |
| Aminomethylpropanol | 6.0 |
| Hydroxypropyl xanthan gum | 0.6 |
| Cetrimonium chloride | 0.1 |
| Preservative | q.s. |
| Water | to 100 |

The pH of the above composition D is approximately 3.4.

## Claims

**1.** Process for semi-permanent straightening and dyeing hair:
wherein the semi-permanent straightening comprises the steps of:

a. optionally washing the hair with a cleansing composition and towel drying,
b. applying to the hair a treatment composition A comprising at least one compound of the general structure

R-CO-R' Formula (I)

and/or a hydrate thereof and/or a salt thereof,
wherein R is selected from hydrogen, COOH, CN, optionally substituted $C_1$-$C_{10}$ alkyl, optionally substituted $C_2$-$C_{10}$ alkenyl, optionally substituted $C_2$-$C_{10}$ alkynyl, optionally substituted $C_3$-$C_{10}$ cycloalkyl, optionally substituted $C_6$-$C_{10}$ aryl or a 5-10-membered, optionally substituted heteroaryl group, wherein the optional substituents of the alkyl group are selected from halogen, hydroxyl, amino and $C_1$-$C_4$ alkoxy, and the optional substituents of the other groups are selected from halogen, hydroxyl, amino, $C_1$-$C_4$ alkyl and $C_1$-$C_4$ alkoxy, and R' is H or COOH, wherein the composition has a pH in the range of 1 to 4 when R' is COOH and in the range of 1 to 6 when R' is H,
c. leaving the composition A on the hair for 1 min to 90 min,
d. optionally rinsing off the hair,
e. optionally drying the hair,
f. heating the hair to a temperature in the range of 130°C to 230°C,
g. optionally rinsing the hair,

wherein the subsequent dyeing carried out immediately after the steps a to g comprises the steps of:

h. optionally washing, towel drying the hair and optionally drying the hair,
i. applying a ready-to-use composition onto the hair consisting of compositions B, C, and D, which are kept

separately until application and mixed to a ready-to-use composition prior to application onto hair at a weight ratio in the range of 1:2:0.1 to 1:1:1 and left on the hair for 1 min to 45 min, and
j. rinsing off the hair with water and/or optionally shampooing the hair, and
k. optionally drying the hair
wherein composition B is an alkaline aqueous composition comprising one or more hair dyes, one or more alkalizing agents and has a pH between 7.5 and 12,
wherein the composition C is an acidic composition having a pH of 2 to 5 and comprising one or more oxidizing compounds, preferably hydrogen peroxide, and
wherein the composition D is a composition comprising

1) one or more carboxylic acids having three or more carboxyl groups and/or their salts, and
2) one or more additional organic acid and/or their salts having one or two carboxyl groups,

wherein composition D comprises the acids 1) and 2) and/or their salts at a total concentration of 10% to 100% by weight, calculated to the total of composition D,
wherein the ready-to-use composition has a pH in the range of 6.5 to 12 and comprises the acids and/or their salts at a total concentration in the range of 1.0% to 10.0% by weight calculated to the total of the ready-to-use composition.

**2.** The process according to claim 1 wherein the carboxylic acid with 3 or more carboxyl groups is selected from citric acid, ethylenediamine tetraacetic acid (EDTA), pyromellitic acid, and glutamate diacetate, wherein the organic acid with one or two carboxyl groups is selected from acetic acid, malic acid, maleic acid, lactic acid, glycolic acid, acetic acid, wherein the composition D preferably comprises the first acid (1) and the second acid (2) at a weight ratio (1)/(2) in the range of 1:0.1 to 1:250, preferably from 1:0.2 to 1:150, and more preferably from 1:0.5 to 1:100 and most preferably 1:50.

**3.** The process according to claims 1 and 2 wherein the composition D is a powder, a dispersion, an emulsion or a solution, preferably it is an aqueous solution.

**4.** The process according to any of the preceding claims wherein the pH of the composition D is in the range from 1 to 5.

**5.** The process according to any of the proceeding claims wherein the carboxylic acid with 3 or more carboxyl groups is EDTA and/or its salts.

**6.** The process according to any of the preceding claims wherein the organic acid with one or two carboxyl group is malic acid and/or its salts.

**7.** The process according to any of the proceeding claims wherein the composition B comprises one or more oxidative dye precursors preferably selected from phenylendiamines, p-aminophenols, and heterocyclic compounds such as diaminopyrazols and substituted pyrimidines, optionally one or more coupling substances preferably selected from resorcinols, m-aminophenols, m-phenylendiamines, pyridines, and naphthols.

**8.** The process according to any of the preceding claims wherein the composition B and/or C and/or D comprise(s) one or more hair direct dye selected from cationic, anionic and nitro dyes or their mixtures.

**9.** The process according to any of the preceding claims wherein the composition(s) B and/or D comprise(s) at least one alkalizing agent selected from ammonia, alkyl- or alkanolamines according to the general structure

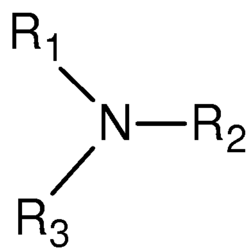

wherein $R_1$, $R_2$, and $R_3$ are same or different H, from $C_1$ to $C_4$, $C_3$ to $C_4$ unsaturated alkyl, $C_3$ to $C_4$ branched alkyl,

$C_1$ to $C_4$ hydroxyl alkyl, $C_3$ to $C_4$ unsaturated hydroxyl alkyl, $C_3$ to $C_4$ branched hydroxyl alkyl, with the condition that at least one of $R_1$, $R_2$, or $R_3$ is different from H, wherein the alkalizing agents preferably selected from ammonia, monoethanolamine, and aminomethylpropanol, and particularly suitable one is aminomethylpropanol.

**10.** The process according to any of the preceding claims **characterized in that** the alkalizing agents are comprised at a concentration range from 1.0% to 10.0% by weight, calculated to the total of each composition.

**11.** The process according to any of the preceding claims wherein the compositions A, B, C or D comprise one or more ingredients selected form fatty alcohols, surfactants selected from anionic, nonionic, cationic and amphoteric ones, ubiquinones, ceramides, reducing agents, organic solvents, silicones such as linear polysiloxanes, aminated silicones, cyclic silicones, arylated silicones, antioxidants, preservatives, amino acids, polyols.

**12.** The process according to any of the proceeding claims wherein the hair is heated with an iron, preferably with a flat iron.

**13.** The process according to any of the preceding claims wherein the composition A comprises glyoxylic acid and/or its hydrates and/or its salts at a concentration range from 1.0% to 40.0% by weight, calculated to the total of the composition A.

**14.** Kit for hair comprising the compositions A, B, C and D as described in the claims 1 to 14.

## Patentansprüche

**1.** Verfahren zum semipermanenten Glätten und Färben von Haaren:

wobei das semipermanente Glätten die Schritte aufweist:

a. gegebenenfalls Waschen der Haare mit einer Reinigungszusammensetzung und Trocknen mit einem Handtuch,
b. Aufbringen einer Behandlungszusammensetzung A auf die Haare, aufweisend mindestens eine Verbindung der allgemeinen Struktur

R-CO-R' Formel (I)

und/oder ein Hydrat davon und/oder ein Salz davon,
wobei R aus Wasserstoff, COOH, CN, gegebenenfalls substituiertem $C_1$-$C_{10}$-Alkyl, gegebenenfalls substituiertem $C_2$-$C_{10}$-Alkenyl, gegebenenfalls substituiertem $C_2$-$C_{10}$-Alkinyl, gegebenenfalls substituiertem $C_3$-$C_{10}$-Cycloalkyl, gegebenenfalls substituiertem $C_6$-$C_{10}$-Aryl oder einer 5-10-gliedrigen, gegebenenfalls substituierten Heteroarylgruppe ausgewählt ist, wobei die gegebenenfalls vorhandenen Substituenten der Alkylgruppe aus Halogen, Hydroxy, Amino und $C_1$-$C_4$-Alkoxy ausgewählt sind und die gegebenenfalls vorhandenen Substituenten der anderen Gruppen aus Halogen, Hydroxy, Amino, $C_1$-$C_4$-Alkyl und $C_1$-$C_4$-Alkoxy ausgewählt sind, und R' H oder COOH ist, wobei die Zusammensetzung einen pH-Wert im Bereich von 1 bis 4 aufweist, wenn R' COOH ist und im Bereich von 1 bis 6 aufweist, wenn R' H ist,
c. Belassen der Zusammensetzung A auf den Haaren für 1 min bis 90 min,
d. gegebenenfalls Ausspülen der Haare,
e. gegebenenfalls Trocknen der Haare,
f. Erwärmen der Haare auf eine Temperatur im Bereich von 130 °C bis 230 °C,
g. gegebenenfalls Ausspülen der Haare,

wobei das anschließende Färben, das unmittelbar nach den Schritten a bis g durchgeführt wird, die Schritte aufweist:

h. gegebenenfalls Waschen, Trocknen der Haare mit einem Handtuch und gegebenenfalls Trocknen der Haare,
i. Aufbringen einer gebrauchsfertigen Zusammensetzung auf die Haare, die aus den Zusammensetzungen B, C und D besteht, welche bis zum Aufbringen getrennt gehalten und vor dem Aufbringen auf die Haare zu einer gebrauchsfertigen Zusammensetzung in einem Gewichtsverhältnis im Bereich von 1:2:0,1 bis

1:1:1 vermischt werden und für 1 min bis 45 min auf den Haaren belassen werden, und
j. Ausspülen der Haare mit Wasser und/oder gegebenenfalls Shampoonieren der Haare, und
k. gegebenenfalls Trocknen der Haare,

wobei die Zusammensetzung B eine alkalische wässrige Zusammensetzung ist, welche einen oder mehrere Haarfarbstoffe aufweist, ein oder mehrere Alkalisierungsmittel aufweist und einen pH-Wert von 7,5 bis 12 aufweist,
wobei die Zusammensetzung C eine saure Zusammensetzung mit einem pH-Wert von 2 bis 5 ist und eine oder mehrere oxidierende Verbindungen, vorzugsweise Wasserstoffperoxid, aufweist und
wobei die Zusammensetzung D eine Zusammensetzung ist, aufweisend:

1) eine oder mehrere Carbonsäuren mit drei oder mehr Carboxylgruppen und/oder deren Salze und
2) eine oder mehrere zusätzliche organische Säuren und/oder deren Salze, welche eine oder zwei Carboxylgruppen aufweisen,

wobei die Zusammensetzung D die Säuren 1) und 2) und/oder deren Salze in einer Gesamtkonzentration von 10 Gewichts-% bis 100 Gewichts-% aufweist, bezogen auf die Gesamtzusammensetzung D,
wobei die gebrauchsfertige Zusammensetzung einen pH-Wert im Bereich von 6,5 bis 12 aufweist und die Säuren und/oder deren Salze in einer Gesamtkonzentration im Bereich von 1,0 Gewichts-% bis 10,0 Gewichts-% aufweist, bezogen auf die gesamte gebrauchsfertige Zusammensetzung.

**2.** Verfahren nach Anspruch 1, wobei die Carbonsäure mit 3 oder mehr Carboxylgruppen aus Citronensäure, Ethylendiamintetraessigsäure (EDTA), Pyromellitsäure und Glutamatdiacetat ausgewählt ist, wobei die organische Säure mit einer oder zwei Carboxylgruppen aus Essigsäure, Äpfelsäure, Maleinsäure, Milchsäure, Glykolsäure, [illegible]sigsäure ausgewählt ist, wobei die Zusammensetzung D vorzugsweise die erste Säure (1) und die zweite Säure (2) in einem Gewichtsverhältnis (1)/(2) im Bereich von 1:0,1 bis 1:250, vorzugsweise von 1:0,2 bis 1:150 und insbesondere von 1:0,5 bis 1:100 und am meisten bevorzugt von 1:50 aufweist.

**3.** Verfahren nach Anspruch 1 und 2, wobei die Zusammensetzung D ein Pulver, eine Dispersion, eine Emulsion oder eine Lösung ist, vorzugsweise eine wässrige Lösung.

**4.** Verfahren nach einem der vorhergehenden Ansprüche, wobei der pH-Wert der Zusammensetzung D im Bereich von 1 bis 5 liegt.

**5.** Verfahren nach einem der vorhergehenden Ansprüche, wobei es sich bei der Carbonsäure mit 3 oder mehr Carboxylgruppen um EDTA und/oder deren Salze handelt.

**6.** Verfahren nach einem der vorhergehenden Ansprüche, wobei es sich bei der organischen Säure mit einer oder zwei Carboxylgruppen um Äpfelsäure und/oder deren Salze handelt.

**7.** Verfahren nach einem der vorhergehenden Ansprüche, wobei die Zusammensetzung B eine oder mehrere Oxidativfarbstoff-Vorstufen, vorzugsweise ausgewählt aus Phenylendiaminen, p-Aminophenolen und heterocyclischen Verbindungen, z.B. Diaminopyrazolen und substituierten Pyrimidinen, und gegebenenfalls ein oder mehrere Kupplungsmittel aufweist, vorzugsweise ausgewählt aus Resorcinolen, m-Aminophenolen, m-Phenylendiaminen, Pyridinen und Naphtholen.

**8.** Verfahren nach einem der vorhergehenden Ansprüche, wobei die Zusammensetzung B und/oder C und/oder D einen oder mehrere Haar-Direktfarbstoffe aufweist/aufweisen, ausgewählt aus kationischen, anionischen und Nitrofarbstoffen oder deren Gemischen.

**9.** Verfahren nach einem der vorhergehenden Ansprüche, wobei die Zusammensetzung(en) B und/oder D mindestens ein Alkalisierungsmittel aufweist/aufweisen, ausgewählt aus Ammoniak, Alkyl- oder Alkanolaminen gemäß der allgemeinen Struktur

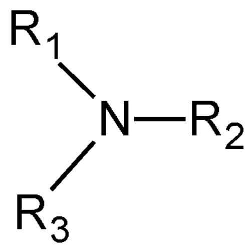

,

wobei $R_1$, $R_2$ und $R_3$ gleich oder verschieden und H, von $C_{1-4}$, ungesättigtes $C_{3-4}$-Alkyl, verzweigtes $C_{3-4}$-Alkyl, $C_{1-4}$-Hydroxyalkyl, ungesättigtes $C_{3-4}$-Hydroxyalkyl, verzweigtes $C_{3-4}$-Hydroxyalkyl sind, mit der Maßgabe, dass mindestens eines aus $R_1$, $R_2$, oder $R_3$ nicht H ist, wobei die Alkalisierungsmittel vorzugsweise aus Ammoniak, Monoethanolamin und Aminomethylpropanol ausgewählt sind und ein besonders geeignetes Aminomethylpropanol ist.

**10.** Verfahren nach einem der vorhergehenden Ansprüche, **dadurch gekennzeichnet, dass** die Alkalisierungsmittel in einem Konzentrationsbereich von 1,0 Gewichts-% bis 10,0 Gewichts-% enthalten sind, bezogen auf die jeweilige Gesamtzusammensetzung.

**11.** Verfahren nach einem der vorhergehenden Ansprüche, wobei die Zusammensetzungen A, B, C oder D einen oder mehrere Inhaltsstoffe aufweisen, ausgewählt aus Fettalkoholen, Tensiden, ausgewählt aus anionischen, nichtionischen, kationischen und amphoteren, Ubichinonen, Ceramiden, Reduktionsmitteln, organischen Lösungsmitteln, Siliconen, z.B. linearen Polysiloxanen, aminierten Siliconen, cyclischen Siliconen, arylierten Siliconen, Antioxidantien, Konservierungsmitteln, Aminosäuren, Polyolen.

**12.** Verfahren nach einem der vorhergehenden Ansprüche, wobei die Haare mit einem Eisen erwärmt werden, vorzugsweise mit einem Plätteisen.

**13.** Verfahren nach einem der vorhergehenden Ansprüche, wobei die Zusammensetzung A Glyoxylsäure und/oder deren Hydrate und/oder deren Salze in einem Konzentrationsbereich von 1,0 Gewichts-% bis 40,0 Gewichts-% aufweist, bezogen auf die Gesamtzusammensetzung A.

**14.** Kit für Haare, aufweisend die Zusammensetzungen A, B, C und D, wie in den Ansprüchen 1 bis 13 beschrieben.

## Revendications

**1.** Procédé de lissage semi-permanent et de coloration des cheveux :

dans lequel le lissage semi-permanent comprend les étapes :

a. de lavage éventuel des cheveux avec une composition de nettoyage et de séchage à la serviette,
b. d'application sur les cheveux d'une composition de traitement A comprenant au moins un composé selon la structure générale

R-CO-R' Formule (I)

et/ou un hydrate de celui-ci, et/ou un sel de celui-ci,
où R est choisi parmi un atome d'hydrogène, un groupe COOH, un groupe CN, un groupe alkyle en $C_1$ à $C_{10}$ éventuellement substitué, un groupe alcényle en $C_2$ à $C_{10}$ éventuellement substitué, un groupe alcynyle en $C_2$ à $C_{10}$ éventuellement substitué, un groupe cycloalkyle en $C_3$ à $C_{10}$ éventuellement substitué, un groupe aryle en $C_6$ à $C_{10}$ éventuellement substitué ou un groupe hétéroaryle de 5 à 10 chaînons éventuellement substitué, où les substituants éventuels du groupe alkyle sont choisis parmi un atome d'halogène, un groupe hydroxyle, un groupe amino et un groupe alcoxy en $C_1$ à $C_4$, et les substituants éventuels des autres groupes sont choisis parmi un atome d'halogène, un groupe hydroxyle, un groupe amino, un groupe alkyle en $C_1$ à $C_4$ et un groupe alcoxy en $C_1$ à $C_4$, et R' est un atome H ou un groupe COOH, où la composition a un pH dans la plage de 1 à

4, lorsque R' est le groupe COOH, et dans la plage de 1 à 6, lorsque R' est l'atome H,
c. de laisser la composition A sur les cheveux pendant 1 min à 90 min,
d. de rinçage éventuel des cheveux,
e. de séchage éventuel des cheveux,
f. de chauffage des cheveux à une température dans la plage de 130 °C à 230 °C,
g. de rinçage éventuel des cheveux,

où la coloration ultérieure effectuée immédiatement après les étapes a à g comprend les étapes :

h. de lavage éventuel, de séchage à la serviette des cheveux et de séchage éventuel des cheveux,
i. d'application sur les cheveux d'une composition prête à l'emploi constituée des compositions B, C, et D, qui sont conservées séparément jusqu'à l'application et mélangées pour donner une composition prête à l'emploi avant l'application sur les cheveux à un ratio pondéral de 1 : 2 : 0,1 à 1 : 1 : 1 et gardée sur les cheveux pendant 1 min à 45 min, et
j. de rinçage des cheveux avec de l'eau et/ou de lavage éventuel des cheveux avec un shampoing, et
k. éventuellement de séchage des cheveux

où la composition B est une composition aqueuse alcaline comprenant un ou plusieurs colorants pour cheveux, un ou plusieurs agents d'alcalinisation et a un pH entre 7,5 et 12,
où la composition C est une composition acide ayant un pH de 2 à 5 et comprenant un ou plusieurs composés oxydants, de préférence, du peroxyde d'hydrogène, et
où la composition D est une composition comprenant

1) un ou plusieurs acides carboxyliques ayant trois ou plus de trois groupes carboxyles, et/ou leurs sels, et
2) un ou plusieurs acides organiques supplémentaires ayant un ou deux groupes carboxyles, et/ou leurs sels,

où la composition D comprend les acides 1) et 2), et/ou leurs sels, à une concentration totale de 10 % à 100 % en poids, calculée par rapport au total de la composition D,
où la composition prête à l'emploi a un pH dans la plage de 6,5 à 12 et comprend les acides, et/ou leurs sels, à une concentration totale dans la plage de 1,0 % à 10,0 % en poids, calculée par rapport au total de la composition prête à l'emploi.

**2.** Procédé selon la revendication 1, dans lequel l'acide carboxylique avec trois ou plus de 3 groupes carboxyles est choisi parmi l'acide citrique, l'acide éthylène diamine tétra acétique (EDTA), l'acide pyroméllitique, et le glutamate di acétate, où l'acide organique avec un ou deux groupes carboxyles est choisi parmi l'acide acétique, l'acide malique, l'acide maléique, l'acide lactique, l'acide glycolique, l'acide acétique, où la composition D comprend de préférence le premier acide (1) et le second acide (2) à un ratio pondéral (1)/(2) dans la plage de 1 : 0,1 à 1 : 250, de préférence de 1 : 0,2 à 1 : 150, et plus préférentiellement de 1 : 0,5 à 1 : 100 et idéalement de 1 : 50.

**3.** Procédé selon les revendications 1 et 2, dans lequel la composition D est une poudre, une dispersion, une émulsion ou une solution, de préférence, c'est une solution aqueuse.

**4.** Procédé selon l'une quelconque des revendications précédentes, dans lequel le pH de la composition D est dans la plage de 1 à 5.

**5.** Procédé selon l'une quelconque des revendications précédentes, dans lequel l'acide carboxylique avec 3 ou plus de trois groupes carboxyles est de l'EDTA, et/ou ses sels.

**6.** Procédé selon l'une quelconque des revendications précédentes, dans lequel l'acide organique avec un ou deux groupes carboxyles est de l'acide malique, et/ou ses sels.

**7.** Procédé selon l'une quelconque des revendications précédentes, dans lequel la composition B comprend un ou plusieurs précurseurs de colorants oxydatifs choisis de préférence parmi les phénylène diamines, les p-amino phénols, et des composés hétérocycliques tels que des diamino pyrazoles et des pyrimidines substituées, éventuellement une ou plusieurs substances de couplage choisies de préférence parmi des résorcinols, des m-amino phénols, des m-phénylène diamines, des pyridines, et des naphtols.

**8.** Procédé selon l'une quelconque des revendications précédentes, dans lequel la composition B et/ou C et/ou D,

comprend/comprennent un ou plusieurs colorants directs des cheveux choisis parmi des colorants cationiques, anioniques et nitro, ou leurs mélanges.

**9.** Procédé selon l'une quelconque des revendications précédentes, dans lequel la/les composition/s B et/ou D comprend/comprennent au moins un agent d'alcalinisation choisi parmi l'ammoniac, des alkyl amines ou alcanol amines selon la structure générale

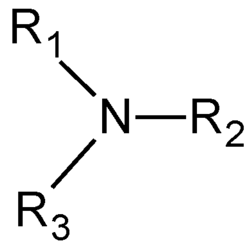

où $R_1$, $R_2$, et $R_3$ sont identiques ou différents d'un atome H, de $C_1$ to $C_4$, un groupe alkyle en $C_3$ à $C_4$ insaturé, un groupe alkyle ramifié en $C_3$ à $C_4$, un groupe alkyle hydroxylé en $C_1$ à $C_4$, un groupe alkyle hydroxylé en $C_3$ à $C_4$ insaturé, un groupe alkyle hydroxylé en $C_3$ à $C_4$ ramifié, à condition qu'au moins un parmi $R_1$, $R_2$, ou $R_3$ est différent de l'atome H, où les agents d'alcalinisation sont de préférence choisis parmi l'ammoniac, la mono éthanolamine, et l'amino méthyl propanol, et un agent particulièrement approprié est l'amino méthyl propanol.

**10.** Procédé selon l'une quelconque des revendications précédentes, **caractérisé en ce que** les agents d'alcalinisation sont compris dans une plage de concentration de 1,0 % à 10,0 % en poids, calculée par rapport au total de chaque composition.

**11.** Procédé selon l'une quelconque des revendications précédentes, dans lequel les compositions A, B, C ou D comprennent un ou plusieurs constituants choisis parmi des alcools gras, des tensioactifs choisis parmi des tensioactifs anioniques, non ioniques, cationiques et amphotères, des ubiquinones, des céramides, des agents de réduction, des solvants organiques, des silicones tels que des polysiloxanes linéaires, des silicones aminés, des silicones cycliques, des silicones arylés, des antioxydants, des conservateurs, des acides aminés, des polyols.

**12.** Procédé selon l'une quelconque des revendications précédentes, dans lequel les cheveux sont chauffés avec un fer, de préférence un fer plat.

**13.** Procédé selon l'une quelconque des revendications précédentes, dans lequel la composition A comprend de l'acide glyoxylique, et/ou ses hydrates, et/ou ses sels, à une plage de concentration de 1,0 % à 40,0 % en poids, calculée par rapport au total de la composition A.

**14.** Kit pour cheveux comprenant les compositions A, B, C et D selon les revendications 1 à 13.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011104282 A
- WO 2012010351 A
- US 20150034119 A
- US 20150037270 A
- WO 2015017768 A
- WO 2014068102 A
- WO 2014067702 A
- WO 9515144 A